# EUROPEAN PATENT APPLICATION

(11) **EP 4 825 336 A1**
(43) Date of publication of application: **21.10.2026**
(21) Application number: 24904418.1
(22) Date of filing: 13.12.2024
(51) Int. Cl.: A61C 13/00, A61C 13/083, A61C 5/30, A61C 5/70, A61K 6/80

(54) **COMPOSITE BULK BLOCK FOR DENTAL PROSTHESIS FABRICATION, MANUFACTURING METHOD THEREFOR, AND PROSTHESIS FABRICATED THEREFROM**

(30) Priority: 15.12.2023 KR 20230183056; 15.12.2023 KR 20230183057; 12.12.2024 KR 20240185094
(71) Applicant: Hass Co., Ltd., Gangwon-do 25452 (KR)
(72) Inventor: KIM, Sung Min, Yongin-si Gyeonggi-do 16903 (KR); KOH, Hwan Soon, Anyang-si Gyeonggi-do 13910 (KR); KIM, Moon Chang, Gangneung-si Gangwon-do 25583 (KR); CHO, Heon Jin, Gangneung-si Gangwon-do 25425 (KR); OH, Jin Woo, Gwangmyeong-si Gyeonggi-do 14259 (KR)
(74) Representative: Gulde & Partner
(86) International application number: PCT/KR2024/096893
(87) International publication number: WO 2025/127853

(57) **Abstract**

Proposed is a composite bulk block for manufacturing a dental prosthesis that includes a glass ceramic matrix and a polymer and in which the glass ceramic matrix is formed of an amorphous glass matrix and crystalline phases dispersed in the glass matrix, the crystalline phases include, as a main crystalline phase, at least one type selected from a leucite crystalline phase and a lithium disilicate crystalline phase, the glass ceramic matrix has an average particle size of 3 to 10pm, and the polymer is included in an amount of 20 to 40 percent by weight with respect to the weight of the entire bulk block and includes an amphoteric ionic group, a method for manufacturing the composite bulk block, and a prosthesis manufactured from the composite bulk block. The composite bulk block for manufacturing a dental prosthesis allows for machining of a restorative material having excellent mechanical properties and an antifouling property.

## Description

### [Technical Field]

The disclosure relates to a composite bulk block for manufacturing a dental prosthesis that can exhibit excellent mechanical strength and an antifouling property, to a method for manufacturing the same, and to a dental prosthesis manufactured therefrom.

### [Background Art]

In the case of prosthetic materials that have been used in the related art, such as porcelain or metal, as the dental industry has developed, issues such as properties or dental esthetics have emerged, so their market share has been gradually decreasing in terms of material. In order to replace them, the market share of materials such as crystallized glass and zirconia has been increasing. Further, in the case of hot-press molding that is a prosthesis manufacturing method that has been used in the related art, there is a problem that the manufacturing time of prostheses is long, so it is difficult to manufacture one-day prostheses currently promoted in the dental market, and thus a transition to CAD/CAM systems occurs. In order to support this transition, the usefulness of a 1:1 machinable material that can be immediately placed after processing without additional heat treatment of crystallized glass has been emphasized in terms of material.

Of course, many materials exhibiting excellent esthetics and high properties are being used due to advances in zirconia and crystallized glass, but, most ceramic materials undergo a crystallization heat-treatment process after machining, so it is difficult to use them as a one-day prosthesis.

Further, in the case of ceramic materials that are currently machined on a 1:1 basis, there is a problem that the machinability is low and chipping occurs in a margin portion (the boundary portion between a prosthesis and a tooth) due to the use of ceramic materials that have already undergone crystallization.

A composite containing both organic and inorganic substances has been developed to solve these problems and meet the current demands of the dental market. A composite is a complementary material in which an organic component suppresses brittleness, which is one of the disadvantages of an inorganic component, and an inorganic component enhances the properties, such as low strength, of an organic component. Further, since a composite has the advantage that it can be subjected to 1:1 machining that enables immediate intraoral placement after processing, technology continues to be developed and new products are being launched. Composites have strength of approximately 150 to 200 MPa, depending on currently commercialized products, and also have the advantage that their machinability is superior to that of existing glass ceramics that can be machined on a 1:1 basis.

In relation to such composites, the inventors, in Korean Patent No. 10-1682542, have disclosed a method of manufacturing a dental block, including: a step of preparing a glass ceramic; a step of forming a ceramic porous body by rendering the glass ceramic porous; a first infiltration step of primarily infiltrating a polymer into the ceramic porous body in a vacuum state by loading the ceramic porous body into a vacuum chamber; and a second infiltration step of secondarily infiltrating a polymer into the ceramic porous body before the primarily infiltrated polymer is completely cured, and characterized by achieving a biaxial flexural strength of 100 to 150 MPa.

The glass ceramic herein is a feldspar-based glass ceramic, specifically containing N₂O 2.0~6.0 percent by weight, SiO₂ 60.0~65.0 percent by weight, K₂O 8.0~15.0 percent by weight, CaO 0.5~3.0 percent by weight, BaO 0.5~2.0 percent by weight, CeO₂ 0.2~1.0 percent by weight, TiO₂ 0~0.5 percent by weight, Al₂O₃ 16.0~19.0 percent by weight for increasing a glass transition temperature and a softening point and enhancing the chemical durability of the crystallized glass, and coloring components 0~1.0 percent by weight that affect coloring, including brightness and chroma, and exhibit fluorescence.

Further, Korean Patent No. 10-1609291 discloses a method of manufacturing a dental block, which includes: a step of crushing glass and then melting the glass at a temperature of 1,400 to 1,800°C; a step of cooling and then crushing the molten glass, and performing a crystallization heat treatment on the crushed glass at 875 to 970°C; a step of re-crushing the crystallized glass subjected to the crystallization heat treatment, and then performing a porous heat treatment on the crystallized glass at a temperature of 700 to 840°C; and a step of infiltrating a polymer into the porous body formed in the crystallized glass subjected to the porous heat treatment, wherein the glass molten at a temperature of 1,400 to 1,800°C contains N₂O 2.0~6.0 percent by weight, SiO₂ 60~65.0 percent by weight, K₂O 8.0~15 percent by weight, CaO 0.5~3.0 percent by weight, BaO 0.5~2.0 percent by weight, CeO₂ 0.2~1.0 percent by weight, TiO₂ over 0 to 0.5 percent by weight, and Al₂O₃ 16.0~19.0 percent by weight.

In detail, the glass herein is also a feldspar-based glass, and the resulting block is disclosed to exhibit a biaxial flexural strength of about 100 to 150 MPa.

On the other hand, Korean Registration No. 10-2122202 proposes a method of manufacturing a composite using chemical bonding between inorganic and organic materials via a silane coupling agent. Specifically, the method includes: a step of adding a thermal initiator to a first mixture obtained by mixing at least two organic materials having different viscosities at 20 to 70°C; a step of surface-treating the inorganic material using a second mixture in which 10 to 14 percent by weight of an acrylic silane coupling agent is mixed in ethanol; a step of mixing the inorganic material surface-treated by the second mixture with the first mixture containing the thermal initiator; and a step of curing the resulting mixture through thermal polymerization at 100 to 150°C.

Further, Korean Patent No. 10-2228118 proposes a dental composite composition including crystallized glass and a curable organic material, in which the crystallized glass has an average crystal size of 50 to 400 nm and from which a dental composite having a biaxial flexural strength of 200 to 300 MPa and a Vickers hardness of 270 to 300 Hv can be provided.

As another example, U.S. Patent No. 7,807,227 discloses a composite material including a porous inorganic-nonmetallic matrix and a second material, and a method for manufacturing the composite material. The method for manufacturing a composite material includes: obtaining a porous inorganic-nonmetallic matrix sintered body by sintering an inorganic-nonmetallic starting material; obtaining a surface-modified product by coating a coupling agent on the surface of the porous inorganic-nonmetallic matrix; infiltrating an organic material into the surface-modified porous inorganic-nonmetallic matrix; and coagulating the organic material, in order to obtain an isotropic composite material, in which the isotropic composite material includes a porous inorganic-nonmetallic matrix having a bending strength of 40 MPa ore more measured in accordance with ISO 6872 and an organic material at least partially filling the pores of the porous inorganic-nonmetallic matrix, and the isotropic composite material has a modulus of elasticity of 25 GPa or more measured in accordance with ISO 10477 and a bending strength of 100 MPa or more measured in accordance with ISO 6872.

Japanese Patent Registration No. 4636514 discloses a dental material that can maintain mechanical strength such as wear resistance and bending strength, color stability, stain resistance, and esthetics over a long period, that is excellent in terms of modulus of bending elasticity and impact strength, and that is also suitable for machining using a low-cost CAD/CAM system, and a method of manufacturing the dental material. The method is a method of manufacturing a dental material by infiltrating a porous ceramics with resin, and includes: (a) molding a mixture into a predetermined shape, the mixture including ceramic powder having an average particle size of 3.0 to 50 µm and containing a network-forming oxide, an intermediate oxide, and a network-modifying oxide, together with a binder; (b) firing the molded mixture and obtaining a porous ceramics block having through-holes; (c) treat the surfaces of the through-holes by infiltrating at least one coupling agent selected from a silane coupling agent, a titanate-based coupling agent, and a zirconium-aluminate-based coupling agent into the through-holes of the porous ceramics block under an ultrasonic and/or reduced-pressure condition; and (d) infiltrating a monomer and/or oligomer containing at least one ethylenically double bond into the through-holes of the coupling-treated porous ceramics block under an ultrasonic and/or reduced-pressure condition, and then polymerizing the monomer and/or oligomer. As a specific example, the ceramic powder used herein is an aluminosilicate ceramic powder in which the network-forming oxides are SiO₂ and B₂O₃, the intermediate oxide is Al₂O₃, and the network-modifying oxide is Na₂O. By infiltrating resin into the through-holes of the porous ceramics, the filler content of the inorganic material can be increased, wear resistance, mechanical strength such as flexural strength, color stability, stain resistance, and esthetics can be maintained over a long period, and stress within the ceramic is alleviated. Accordingly, it is possible to provide a dental material that exhibits excellent properties in terms of modulus of bending elasticity and impact strength, and the dental material is also described as being suitable for a CAD/CAM system.

Meanwhile, it is known that a zwitterionic material (also referred to as a bipolar ionic material and an amphoteric ionic material) is used with a variety of dental materials and contributes to an antifouling effect of blocking adsorption of proteins and bacteria. As an example, mention may be made of a dental polymeric composition that contains an aggregate having polymerizable monomers and a zwitterionic group as a side chain, as described in Japanese Patent Publication No. 2007-217516. Here, by using an aggregate having a zwitterionic group as a side chain, a high effect of suppressing the adsorption of proteins and plaque is shown.

Further, Japanese Patent Publication No. 2014-009219 discloses a dental adhesive composition in which an aggregate having a zwitterionic group as a side chain is used together with water, and describes that the dental adhesive composition is useful as a dental pretreatment agent, an adhesive, and an adhesive composite resin.

In addition, there is also disclosed a method including a step of exposing a superhydrophilic titanium surface having bioactivity by removing contaminants adsorbed to and stabilized on a titanium oxide film, and a step of forming a uniform coating film by applying a mixed solution including a pH buffering agent and an organic amphoteric substance having a sulfonic group, that is, a technique relating to a dental implant made of titanium or a titanium alloy, in which a coating layer of a mixed solution containing i) an organic pH buffering substance and/or an inorganic pH buffering substance and ii) an organic amphoteric substance having a sulfonic group is formed on the rough surface pretreated so that contaminants are removed (Korean Patent No. 10-1405859).

The inventors have continuously sought an approach for, in a resin-ceramic composite, enabling a prosthesis itself to exhibit an ability to suppress attachment of intra-oral contaminants, in addition to implementing the appearance and functional performance of teeth, and have previously filed an application in relation thereto (Korean Patent Application No. 10-2021-0055575).

### [Disclosure]

### [Technical Problem]

An objective of the present disclosure is to provide a composite bulk block for manufacturing a dental prosthesis, which has improved mechanical strength, can be used not only for inlays and onlays but also as artificial posterior teeth, and can exhibit an attachment-suppression ability against intra-oral contaminants.

Further, an objective of the present disclosure is to provide a method for manufacturing a composite bulk block for manufacturing a prosthesis, the bulk block being capable of exhibiting an attachment-suppression ability against intra-oral contaminants.

Further, an objective of the present disclosure is to provide a dental prosthesis manufactured from a composite bulk block for manufacturing a dental prosthesis having the features described above.

### [Technical Solution]

The present disclosure provides a composite bulk block for manufacturing a dental prosthesis, the composite bulk block including a glass ceramic matrix and a polymer, wherein the glass ceramic matrix is composed of an amorphous glass matrix and a crystalline phase dispersed in the glass matrix, the crystalline phase includes at least one selected from a leucite crystalline phase and a lithium disilicate crystalline phase as a main crystalline phase, the polymer is included by 20 to 40 percent by weight with respect to a total weight of the bulk block, the glass ceramic matrix has an average particle size of 3 to 10µm, and the polymer includes an amphoteric ionic group.

According to a preferred embodiment of the present invention, wherein the amphoteric ionic group is derived from at least one selected from a group composed of 2-methacryloyloxyethyl phosphorylcholine (MPC), sulfobetaine methacrylate (SBMA), (1,2-dimyristoyl-sn-glycero-3-phosphatidylcholine (DMPC), 3-dimethylsulfoniopropanoate (DMSP), trigonelline, ectoine, betaine, N-(2-methacryloyloxy)ethyl-N,N-dimethylammonio propanesulfonate (SPE), N-(3-methacryloylimino)propyl-N,N-dimethylammonio propanesulfonate (SPP), carboxybetaine methacrylate (CBMA), and 3-(2'-vinyl-pyridinio)propanesulfonate (SPV), wherein the amphoteric ionic group is derived from at least one selected from a group composed of 2-methacryloyloxyethyl phosphorylcholine (MPC) and sulfobetaine methacrylate (SBMA), wherein the amphoteric ionic group is derived from 2-methacryloyloxyethyl phosphorylcholine (MPC) and sulfobetaine methacrylate (SBMA).

According to a preferred embodiment of the present invention, wherein the amphoteric ionic group is derived from the use of a mixture of 2-methacryloyloxyethyl phosphorylcholine (MPC) and sulfobetaine methacrylate (SBMA) in an amount of 0.1 to 0.45 percent by weight with respect to the total composition of the composite bulk block.

According to an embodiment of the present invention, wherein the glass ceramic matrix is included in an amount of 74.5 to 77.9 percent by weight.

According to an embodiment of the present invention, wherein the polymer includes a cured product of a curable organic material selected from (meth)acrylate-based monomers and oligomers including an unsaturated double bond.

According to a preferred embodiment of the present invention, wherein the curable organic material is at least one selected from a group composed of hydroxyethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyl dimethacrylate (BPDM), n-tolylglycine-glycidyl methacrylate (NTGE), polyethylene glycol dimethacrylate (PEG-DMA), and oligocarbonate dimethacrylic esters.

According to an embodiment of the present invention, wherein the composite bulk block for manufacturing a dental prosthesis has biaxial flexural strength of 220 to 280 MPa.

According to an embodiment of the present invention, wherein the glass matrix includes SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent.

According to an embodiment of the present invention, wherein the glass ceramic is produced by a method comprising: preparing glass powder having a maximum average particle size within 300µm by melting a glass composition including SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent, by obtaining a glass molded body of assembly size by water-quenching the molten glass, and by primarily crushing the glass molded body; performing crystallization heat treatment on the glass powder in a furnace from room temperature to a maximum temperature of 755~810°C for 30 minutes to 6 hours; producing glass ceramic powder having an average particle size within 3 to 10µm by crushing the crystallization heat-treated powder; and molding the glass ceramic powder into a predetermined shape.

Also, the present invention, a method for manufacturing a composite bulk block for manufacturing a dental prosthesis, the method comprising: preparing crystallizable glass powder having a maximum average particle size within 300µm or less; performing crystallization heat treatment on the crystallizable glass powder in a furnace from room temperature to a maximum temperature of 755~810°C for 30 minutes to 6 hours; producing glass ceramic powder having a maximum average particle size of 3 to 10µm by crushing the crystallization heat-treated powder; and molding a glass ceramic matrix by molding the glass ceramic powder into a predetermined shape.

In the method for manufacturing a composite bulk block for fabricating a dental prosthesis according to the present invention, wherein the polymer is included in an amount of 20 to 40 percent by weight in the entire composite bulk block.

In the method for manufacturing a composite bulk block according to a preferred embodiment, in terms of providing an antifouling property, wherein the polymer includes an amphoteric ionic group.

In a specific embodiment, wherein the amphoteric ionic group is derived from at least one selected from a group composed of 2-methacryloyloxyethyl phosphorylcholine (MPC), sulfobetaine methacrylate (SBMA), (1,2-dimyristoyl-sn-glycero-3-phosphatidylcholine (DMPC), 3-dimethylsulfoniopropanoate (DMSP), trigonelline, ectoine, betaine, N-(2-methacryloyloxy)ethyl-N,N-dimethylammonio propanesulfonate (SPE), N-(3-methacryloylimino)propyl-N,N-dimethylammonio propanesulfonate (SPP), carboxybetaine methacrylate (CBMA), and 3-(2'-vinyl-pyridinio)propanesulfonate (SPV), wherein the amphoteric ionic group is derived from at least one selected from a group composed of 2-methacryloyloxyethyl phosphorylcholine (MPC) and sulfobetaine methacrylate (SBMA), wherein the amphoteric ionic group is derived from 2-methacryloyloxyethyl phosphorylcholine (MPC) and sulfobetaine methacrylate (SBMA).

In a specific embodiment, wherein the amphoteric ionic group is derived from the use of a mixture of 2-methacryloyloxyethyl phosphorylcholine (MPC) and sulfobetaine methacrylate (SBMA) in an amount of 0.1 to 0.45 percent by weight with respect to the total composition of the composite bulk block.

In the method for manufacturing a composite bulk block according to the present invention, wherein the glass ceramic matrix is included in an amount of 74.5 to 77.9 percent by weight.

In an embodiment of the present invention, wherein the polymer includes a cured product of a curable organic material selected from (meth)acrylate-based monomers and oligomers including an unsaturated double bond.

In a specific embodiment, wherein the curable organic material is at least one selected from a group composed of hydroxyethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyl dimethacrylate (BPDM), n-tolylglycine-glycidyl methacrylate (NTGE), polyethylene glycol dimethacrylate (PEG-DMA), and oligocarbonate dimethacrylic esters.

In the method for manufacturing a composite bulk block according to a preferred embodiment of the present invention, wherein the crystallizable glass powder is obtained from a glass composition including SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent, wherein the crystallizable glass powder is obtained by melting a glass composition, obtaining a glass molded body of assembly size by water quenching the molten glass, and primarily crushing the glass molded body.

Additionally, in another embodiment of the present invention, a prosthesis processed from the composite bulk block of the embodiment, the prosthesis comprising a glass ceramic matrix and a polymer, wherein the glass ceramic matrix is composed of an amorphous glass matrix and a crystalline phase dispersed in the glass matrix, the crystalline phase includes at least one selected from a leucite crystalline phase and a lithium disilicate crystalline phase as a main crystalline phase, the polymer is included in an amount of 20 to 40 percent by weight with respect to weight of the entire bulk block, the glass ceramic matrix has an average particle size of 3 to 10µm, and the polymer includes an amphoteric ionic group.

### [Advantageous Effects]

The composite bulk block according to the present disclosure has improved mechanical strength, can be used not only for inlays and onlays but also as artificial posterior teeth, and can exhibit an attachment-suppression ability against intra-oral contaminants.

### [Description of Drawings]

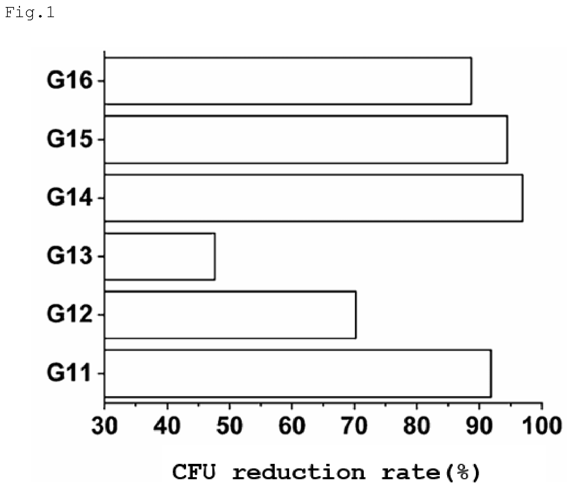

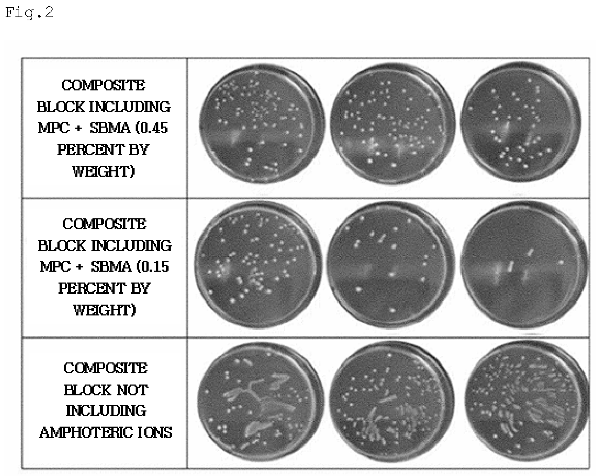

FIGS. 1 to 2 are assessment results for an antifouling property of composite bulk blocks according to the present disclosure; FIG. 1 is a reduction-rate graph evaluating CFU decrease ratios of six experimental groups compared with a control group, and FIG. 2 is a photograph comparing two experimental groups of the experimental groups with the control group.

### [Mode for Invention]

Aspects of the present disclosure that were described above and will be added will be made clearer through exemplary embodiments that are described with reference to the accompanying drawings. Hereinafter, such embodiments of the present disclosure are described in detail so that those skilled in the art can easily understand and achieve the present disclosure.

The present disclosure provides a composite bulk block for manufacturing a dental prosthesis that includes a glass ceramic matrix and a polymer and in which the glass ceramic matrix is formed of an amorphous glass matrix and crystalline phases dispersed in the glass matrix, the crystalline phases include, as a main crystalline phase, at least one type selected from a leucite crystalline phase and a lithium disilicate crystalline phase, the glass ceramic matrix has an average particle size of 3 to 10µm, the polymer is included in an amount of 20 to 40 percent by weight with respect to the weight of the entire bulk block, and the polymer includes an amphoteric ionic group.

In the above and following descriptions, the term "main crystalline phase" is defined as a crystalline phase accounting for at least 50 percent by weight of the total crystalline phases, and the term "additional crystalline phase" may be defined as the remaining crystalline phase(s) other than the main crystalline phase of the total crystalline phases.

The content of the crystalline phase can be determined by X-ray diffraction analysis. For example, in a specimen composed of two crystalline phases, a and b, the proportion Fₐ of the crystalline phase a can be quantitatively represented by the following Mathematical expression 1. [Mathematical expression 1] ${F}_{a} = \frac{1}{1 + K \left(\frac{{I}_{b}}{{I}_{a}}\right)}$

This value can be obtained by measuring the intensity ratio of the two crystalline phases and determining the integer K. K is the absolute intensity ratio Iₒₐ/I_{ob} of the two pure crystalline phases, and is determined by measuring a standard material.

In the above and following descriptions, the term "main crystalline phase" may be defined as that determined on the basis of the content calculated by such a method.

In the above and following descriptions, the composite bulk block is not limited in shape, and may include, for example, bulk bodies of various forms such as a block form, a disk form, an ingot form, and a cylinder form.

The composite bulk block according to the present disclosure includes a glass ceramic as a ceramic in consideration of the refractive index difference between the monomer material forming the polymer and the ceramic. In particular, a lithium disilicate-based glass ceramic or a leucite-based glass ceramic may be preferable in terms of esthetics, and a lithium disilicate-based glass-ceramic may be most preferable.

In the composite bulk block according to the present disclosure, the glass ceramic matrix may preferably have an average particle size of 3 to 10µm, more preferably 3 to 5µm, in terms of enhancing mechanical properties, and the composite bulk block including a glass ceramic matrix having the above average particle size may be desirable in that it can exhibit mechanical properties sufficient for application not only to inlays and onlays but also to posterior crowns after being machined and applied as a prosthesis.

Meanwhile, it may be preferable that the polymer present in the composite bulk block is included by 20 to 40 percent by weight with respect to the total weight of the bulk block. When the polymer is included by less than 20 percent by weight with respect to the total weight of the bulk block, low machinability may occur due to the brittleness of the ceramic, which may be disadvantageous for use. When the polymer is included by exceeding 40 percent by weight, problems such as fracture or wear may occur due to excessively low properties. According to an embodiment of the present invention, wherein the glass ceramic matrix is included in an amount of 74.5 to 77.9 percent by weight.

Meanwhile, in the composite bulk block according to the present disclosure, the polymer may include an amphoteric ionic group.

An amphoteric ionic group may be defined as a functional group of amphoteric ions or zwitterions, which refer to molecules that are electrically neutral by possessing both positive and negative charges in chemistry, and as one example of the present disclosure, it may be derived from at least one amphoteric compound selected from a group composed of 2-methacryloyloxyethyl phosphorylcholine (MPC), sulfobetaine methacrylate (SB), 1,2-dimyristoyl-sn-glycero-3-phosphatidylcholine (DMPC), DMSP (3-dimethylsulfoniopropanoate), 3-dimethylsulfoniopropanoate (DMSP), trigonelline, ectoine, betaine, N-(2-methacryloyloxy)ethyl-N,N-dimethylammonio propanesulfonate (SPE), N-(3-methacryloylimino)propyl-N,N-dimethylammonio propanesulfonate (SPP), carboxybetaine methacrylate (CBMA), and 3-(2'-vinyl-pyridinio)propanesulfonate (SPV).

For example, 2-Methacryloyloxyethyl phosphorylcholine (MPC) is attracting attention as a bio-friendly and eco-friendly material that mimics the hydrophilic functional group of phosphatidylcholine (PC), which is a component of the biological double membrane phospholipid. In addition, since MPC includes the above-described hydrophilic functional group, it may form a stable hydration shell with high hydrophilicity, thereby having excellent anti-fouling function.

Sulfobetaine methacrylate (SBMA) includes sulfobetaine having a sulfonate functional group instead of the above-described phosphate group of phosphatidylcholine, it is bio-friendly and has high potential for in vivo application, similar to MPC, and is a zwitterion having both a sulfonate anion group and an ammonium cation group in one molecule. Moreover, SBMA also has an antifouling function and may be easily and conveniently synthesized compared to PC-based polymers, and thus may be easily used for more commercial applications.

Although the above-described zwitterionic materials MPC and SBMA may show some degree of difference, most dipolar polymers may inhibit non-specific adsorption in aqueous solution through hydrophobic or electrostatic attraction. In addition, the addition of a zwitterionic material as an additive may inhibit suppress thermal and chemical denaturation of the protein. In particular, the above-described MPC and SBMA may be used more stably in vivo by mimicking in vivo components.

In this regard, the amphoteric ionic group may be derived from at least one selected from a group composed of 2-methacryloyloxyethyl phosphorylcholine (MPC) and sulfobetaine methacrylate (SBMA).

Most preferably, the amphoteric group may be derived from 2-methacryloyloxyethyl phosphorylcholine (MPC) and sulfobetaine methacrylate (SBMA).

When amphoteric ionic groups derived from MPC and SBMA are both included, the ratio of MPC to SBMA may be set within a weight ratio of 1:3 to 3:1. In this case, when two or more types of amphoteric ionic materials are included in the specific ratio described above, the antifouling property of the composite bulk block tends to be more improved compared to when each is used alone. Most preferably, the weight ratio of MPC and SBMA is 1:1.

In introducing such an amphoteric ionic group into the composite bulk block, the content thereof may preferably be included within an appropriate range that can exhibit an antifouling property without impairing mechanical properties. In this case, the content may preferably be 0.1 to 0.45 percent by weight with respect to the weight of the amphoteric compound in the total composition of the composite bulk block, and more preferably, a mixture of MPC and SBMA may be used in an amount of not less than 0.15 percent by weight and less than 0.45% by weight.

Meanwhile, in the dental bulk block of the present disclosure, a polymer may be bonded to a glass ceramic matrix by silane bonding.

Such silane bonding can be made possible through surface treatment of the glass ceramic matrix, and specifically, it can be made possible by treating the surface of the glass ceramic with an organofunctional silane compound having an ethylenically unsaturated double bond and then bonding a polymer thereto.

More specifically, the organofunctional silane may be at least one selected from a group consisting of methacryloxyalkylene trialkoxysilane, 3-methacryloxypropyl trimethoxysilane, and 3-methacryloxypropyl triethoxysilane, but is not limited thereto.

As an example of such a treatment method, the method described in Korean Patent No. 10-1609291, the method described in Korean Patent No. 10-1682542, the method described in Korean Patent No. 10-2122202, or the method described in Korean Patent No. 10-2228118 may be used.

The polymer contained in the composite bulk block according to the present disclosure may be a cured product of a curable organic material selected from (meth) acrylate-based monomers and oligomers including an unsaturated double bond. As a specific example, the curable organic material may be at least one selected from the group consisting of hydroxyethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyl dimethacrylate (BPDM), n-tolylglycine-glycidyl methacrylate (NTGE), polyethylene glycol dimethacrylate (PEG-DMA), and oligocarbonate dimethacrylic esters.

Among such monomers and/or oligomers, for example, in the case of UDMA or Bis-GMA, due to their high viscosity, they can be mixed with TEGDMA having low viscosity in a mass ratio of 5:5 to 6:4, but the present disclosure is not limited thereto.

Curable organic materials exhibit shrinkage curing during polymerization, and an effect of minimizing changes in properties caused by shrinkage curing can also be obtained by organofunctional silane described above.

Surface treatment of crystallized glass using such an organofunctional silane can, of course, be performed using a solution in which organofunctional silane is diluted in ethanol, in consideration of the particularity of dental composites.

When a composite bulk block is manufactured by treating the surface of a glass ceramic with an organofunctional silane in this manner and bonding it with a polymer, it serves to increase the volume % of an inorganic material in the composite overall, whereby it is possible to enhance the biaxial flexural strength and hardness.

Meanwhile, an initiator may be included to cure a curable organic material by performing crosslinking in the form of a polymer, and a photoinitiator and a thermal initiator may be exemplified as the initiator. In the present disclosure, a preferred initiator is a thermal initiator, and a composite having superior properties can be obtained when heat-polymerization is performed including a thermal initiator, compared to when light-polymerization is performed including a photoinitiator.

Various compounds known in the art can be used as a thermal initiator, and, known peroxides such as dibenzoyl peroxide, dilauroyl peroxide, tert-butyl peroxy-2-ethylhexanoate, or tert-butyl perbenzoate may be exemplified, but the present disclosure is not limited thereto.

In order to obtain the composite bulk block for manufacturing a dental prosthesis according to the present disclosure described above, preferably, a glass matrix may contain SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent.

This glass composition undergoes nucleation and crystal growth heat treatment for crystallization, thereby precipitating crystal phases within an amorphous glass matrix, and the glass matrix undergoes nucleation and crystal growth at a temperature ranging from 500°C to 880°C. That is, nucleation starts at a minimum temperature of 500°C, and crystal growth occurs during heating, and this crystal growth shows the lowest light transmittance in use for a dental prosthesis at a maximum temperature of 880°C. That is, the light transmittance gradually decreases from the temperature at which crystals start to grow up to a maximum temperature of 880°C. Considering this crystal growth, crystals can be grown to an extent that satisfies high strength while ensuring machinability, which can be useful for the glass ceramic matrix of the composite bulk block according to the present disclosure.

From this perspective, the glass ceramic constituting the dental composite block according to the present closure may be obtained through: a step of preparing glass powder having a maximum average particle size within 300 µm by melting a glass composition containing SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt% and P₂O₅ 2.0~6.0 wt%, by obtaining a glass molded body of assembly size by water-quenching the molten glass, and by primarily crushing the glass molded body; a step of performing crystallization heat treatment on the glass powder in a furnace from room temperature to a maximum temperature of 755~810°C for 30 minutes to 6 hours; a step of producing glass ceramic powder having an average particle size of 3 to 10µm, and more preferably 3 to 5µm by crushing the crystallization heat-treated powder; and a step of molding the glass ceramic powder into a predetermined shape.

In a specific embodiment for obtaining the glass ceramic constituting the composite bulk block of the present disclosure, first, a glass composition containing SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt% and P₂O₅ 2.0~6.0 wt% is weighed and mixed.

For the glass composition, Li₂CO₃ may be added instead of Li₂O, and the carbon (C) component of Li₂CO₃, i.e., carbon dioxide (CO₂), is released as a gas and escapes during the glass melting process. Further, in the case of alkali oxides, K₂CO₃ and Na₂CO₃ may be added instead of K₂O and Na₂O, respectively, and the carbon (C) components of K₂CO₃ and Na₂CO₃, i.e., carbon dioxide (CO₂), are released as a gas and escape during the glass melting process.

A dry mixing process is used for mixing, and a ball milling process, etc. may be used as the dry mixing process. In detail, in the ball milling process, a starting material is loaded into a ball milling machine, and the ball milling machine is rotated at a predetermined speed, thereby mechanically crushing and uniformly mixing the starting material. Balls made of a ceramic material such as zirconia or alumina may be used as the balls used in the ball milling machine, and the balls may all have the same size or at least two different sizes. The size of the balls, milling time, and the rotational speed per minute of the ball milling machine are adjusted in consideration of the target particle size. For example, considering the particle size, the size of the balls may be set in the range of about 1 mm to 30 mm, and the rotational speed of the ball milling machine may be set in the range of about 50 to 500 rpm. Ball milling is preferably performed for 1 to 48 hours in consideration of the target particle size, etc. The starting material is crushed into fine particles by ball milling, and is uniformly mixed while having a uniform particle size.

The mixed starting material is loaded into a melting furnace, and the furnace containing the starting material is heated, whereby the starting material is melted. Here, melting refers to the change of the starting material from a solid state to a material state having the viscosity of a liquid state. The melting furnace is preferably made of a material having a high melting point, high strength, and a low contact angle to suppress adhesion of a molten material, and for this purpose, it is preferable that the melting furnace is made of materials such as platinum (Pt), diamond-like carbon (DLC), or chamotte, or that the surface of the melting furnace is coated with materials such as platinum (Pt) or diamond-like carbon (DLC).

Melting is preferably performed at atmospheric pressure at a temperature of 1,400 to 2,000°C for 1 to 12 hours. If the melting temperature is less than 1,400°C, the starting material may not be completely melted, and if the melting temperature exceeds 2,000°C, excessive energy consumption is required, so it is uneconomical. Therefore, it is preferable to perform melting at a temperature within the above range. Further, if the melting time is too short, the starting material may not be sufficiently melted, and if the melting time is too long, excessive energy consumption is required, so it is uneconomical. The heating rate of the melting furnace is preferably in the range of about 5°C/min to 50°C/min. If the heating rate of the melting furnace is too slow, it takes a long time, which results in low productivity, and if the heating rate of the melting furnace is too fast, the rapid temperature increase may cause a large amount of volatilization of the starting material, which may deteriorate the properties of the crystallized glass. Therefore, it is preferable to raise the temperature of the melting furnace at the heating rate in the range described above. Melting is preferably performed in an oxidizing atmosphere such as oxygen (O₂) or air.

In order to crush the molten glass into a desired shape and size, a glass molded body of assembly size is obtained by water-quenching the molten glass, and the glass molded body is primarily crushed, thereby preparing glass powder having a maximum average particle size of 300 µm or less.

The glass powder obtained in this manner is transferred to a crystallization heat-treatment firing furnace, and the intended crystallization heat-treated powder is produced.

In this case, the crystallization heat treatment is performed from room temperature to a maximum temperature of 755 to 810°C for 30 minutes to 6 hours in the furnace, whereby crystallization heat-treated powder having only pure lithium disilicate as a crystalline phase with a crystal size of 0.01 to 1.0 µm can be obtained.

Next, the crystallization heat-treated powder is crushed, thereby producing glass ceramic powder having an average particle size of 3 to 10µm, and more preferably 3 to 5µm.

Finally, a glass ceramic matrix can be obtained through a step of molding the glass ceramic powder into a predetermined shape.

Meanwhile, in the step of molding the glass ceramic powder into a predetermined shape, the molding conditions may also affect the mechanical properties, and as the molding pressure increases and the heat treatment time becomes longer, the mechanical properties may exhibit improvement in mechanical properties.

However, if, in the molding conditions, a molding pressure or a heat treatment time become excessively high or long, the glass ceramic matrix and the polymer may not be sufficiently mixed, which may result in deterioration of mechanical properties. In consideration of this, it is more preferable that the molding pressure is 15 to 25 tons and the heat treatment time is 5 to 7 hours. It should be understood that such molding load or heat treatment time may, of course, be appropriately adjusted within the range described above, depending on the shape or area of the intended composite bulk block.

By using the molded product obtained as described above as a glass ceramic matrix, and by utilizing the method described in Korean Patent No. 10-1609291, the method described in Korean Patent No. 10-1682542, the method described in Korean Patent No. 10-2122202, or the method described in Korean Patent No. 10-2228118, it is possible to manufacture a dental bulk block that includes the glass ceramic matrix and the polymer according to the present disclosure and in which the glass ceramic matrix is composed of an amorphous glass matrix and a crystalline phase dispersed in the glass matrix, the crystalline phase includes at least one selected from a leucite crystalline phase and a lithium disilicate crystalline phase as a main crystalline phase, the glass ceramic matrix has an average particle size of 3 to 10µm, and the polymer is included by 20 to 40 percent by weight with respect to the total weight of the bulk block, and the polymer includes an amphoteric ionic group.

In some cases, a vacuum may be additionally applied in consideration of uniform dispersion of the polymer within the composite bulk block, and the degree of vacuum may also affect the mechanical properties. When the vacuum pressure is about 10⁻² to 10⁻³ torr, it may be preferable in terms of mechanical properties, but it is not limited thereto.

Meanwhile, in the manufacture of the composite bulk block, the curing temperature and time may be appropriately adjusted in consideration of the curing characteristics of the polymer, and the mechanical properties may also be affected by the curing time, that is, the mechanical properties may be improved as the curing time increases.

The machined prosthesis includes a glass ceramic matrix and a polymer, in which the glass ceramic matrix includes an amorphous glass matrix and crystalline phases dispersed in the amorphous glass matrix, the crystalline phases include at least one selected from a leucite crystalline phase and a lithium disilicate crystalline phase as the main crystalline phase, the polymer is included in an amount of 20 to 40 percent by weight relative the weight of the entire bulk block, the glass ceramic matrix has an average particle size of 3 to 10µm, and the polymer includes an amphoteric ionic group. As a result, the prosthesis is useful not only for inlays and onlays but also for posterior crowns because the mechanical properties are excellent. Further, it can exhibit the effect of preventing secondary caries caused by the prosthesis by suppressing bacterial adhesion.

In relation to the composite bulk block for manufacturing a dental prosthesis and the method for manufacturing the composite bulk block described above, the results confirming the effect of the average particle size of the glass ceramic matrix on the mechanical properties of the composite bulk block are shown in Table 1 below. The average particle size herein refers to the average particle size of the glass ceramic powder used to form the glass ceramic matrix, and was calculated using SEM.

In this case, the composite bulk block was manufactured as a composite bulk block with the same composition through the same process.

**[Table 1]**

| Specimen | Average Particle Size of Glass Ceramic Matrix (µm) | Biaxial Flexural Strength of Composite Block (MPa) | Three-Point Flexural Strength of Composite Block (MPa) |
|---|---|---|---|
| 1 | 12.8 | 207.66±7.45 | 184.25±3.45 |
| 2 | 4.6 | 251.78±9.67 | 227.52±1.24 |

In the foregoing and following descriptions, the biaxial flexural strength is defined as a value measured in accordance with ISO 4049. Further, the three-point flexural strength is defined as a value measured in accordance with ISO 6872.

From the contents of Table 1, it can be seen that in the composite bulk block, the mechanical properties vary depending on the particle size of the glass ceramic matrix. In particular, it can be seen that when the average particle size of the glass ceramic matrix is 3 to 10µm, the mechanical properties of the composite block are further improved, and this is preferable.

Until now, hybrid products for dental CAD/CAM have been mainly used for inlays and onlays due to insufficient mechanical properties. In the case of the composite block according to the present disclosure, it can be predicted that it may be applicable to posterior crowns due to its excellent mechanical properties.

Meanwhile, as described above, the composite bulk block according to the present disclosure includes an amphoteric ionic group in the polymer, and in order to determine whether the amphoteric ionic group affects the mechanical properties of the composite bulk block, a comparative block was manufactured without an amphoteric ionic group while keeping all other compositions and processes identical, and then compared. As a result, as shown in Table 2, it was confirmed that the inclusion of an amphoteric ionic group in the composite bulk block does not have a negative effect on mechanical properties. However, if the content is excessive, it may reduce the mechanical properties and it may be difficult to achieve a level of strength sufficient for application to posterior crowns, and thus it can be seen that it is necessary to control the content of a compound derived from an amphoteric ionic group in the composite block.

**[Table 2]**

| Specimen | Average Particle Size of Glass Ceramic Matrix (µm) | Content of Amphoteric Compound in Composition (% by weight) | Biaxial Flexural Strength of Composite Block (MPa) | Three-Point Flexural Strength of Composite Block (MPa) |
|---|---|---|---|---|
| 1 | 4.6 | 0.15 | 251.78±9.67 | 227.52±1.24 |
| 2 | 4.6 | 0 | 253.81±28.51 | 227.55±5.08 |

Meanwhile, in the composite block according to the present disclosure, an experiment was conducted to examine the effect on manifestation of an antifouling property of the composite block depending on the type and content of amphoteric ions.

In this case, a composite block that does not include amphoteric ions, as shown in Sample 2 of Table 5 was prepared as a control group. As for an experimental group, composite blocks including amphoteric compounds were prepared, as shown in Sample 2 of Table 1 and Sample 1 of Table 2. Specifically, the amphoteric compounds were MPC alone, SBMA alone, and a mixture of MPC and SBMA, and a total of six composite bulk block specimens including 0.15 percent by weight and 0.45% by weight, which is the content of the amphoteric compounds, in the entire composition for manufacturing the composite bulk blocks were prepared.

A summary of the specific experimental groups is shown in Table 3 below.

**[Table 3]**

| classification | amphoteric compounds | amphoteric compounds content(wt%) |
|---|---|---|
| G11 | MPC | 0.15 |
| G12 | MPC | 0.45 |
| G13 | SBMA | 0.15 |
| G14 | SBMA | 0.45 |
| G15 | MPC+SBMA | 0.15 |
| G16 | MPC+SBMA | 0.45 |

| | | |
|---|---|---|
| annotation) MPC:2-methacryloxyethyl phosphorylcholine, SBMA: sulfobetaine methacrylate | | |

The method of evaluating an antifouling property is a method of measuring the Colony Forming Unit (CFU), and the specific procedure is as follows.
1) The surface of rectangular specimens having a width of 10 mm, a length of 10 mm, and a height of 2 mm was coated with artificial saliva containing mucin for 4 hours in consideration of clinical conditions.
2) All groups with surfaces treated with artificial saliva were inoculated with Streptococcus mutans (S. mutans) at 1 × 10⁶ CFU/mL and incubated at 37 °C for 72 hours.
3) After incubation at 37 °C for 72 hours, all groups were washed three times with PBS solution and the biofilm formed on the surface was detached by vortexing and sonication.
4) It was diluted 10⁻⁵-fold, spread onto a BHI agar plate, and incubated at 37 °C for 24 hours.
5) The colony-forming unit (CFU) of bacteria formed on the agar plate after incubation for 24 hours was measured.

4) The reduced CFU values of the experimental groups compared to the control group were calculated as a percentage.

The result is as shown in FIG. 1. Further, a photograph of the related culture solution is shown in FIG. 2

From the result shown in FIG. 1, it can be seen that all the composite blocks according to the present disclosure including an amphoteric ionic group exhibit an antifouling property. Specifically, in terms of only the CFU reduction rate, the case where SBMA alone was included at 0.45 percent by weight showed the best result. However, considering the mechanical properties and economic feasibility of a composite bulk block, it can be said that a mixture of MPC and SBMA is preferable, and in particular, the case where MPC and SBMA were mixed and included at 0.15 percent by weight in the entire composition showed the most desirable result.

The composite block according to the above embodiments of the present disclosure specifically has the composition as shown in Table 4 below, but the present disclosure is of course not limited to an example of the compositions.

**[Table 4]**

| composition | content(wt%) |
|---|---|
| glass ceramic matrix | 74.5~77.9 |
| UDMA | 12.3~14.5 |
| TEGDMA | 7.5~8.4 |
| BPO | 0.07~0.08 |
| amphoteric compounds | 0.10~0.45 |

| | |
|---|---|
| annotation) UDMA: diurethanedimethacrylate, TEGDMA: Triethylene glycoldimethacrylate, BPO: Benzoyl Peroxide | |

Although the present disclosure has been described with reference to embodiments, those are only examples and may be changed and modified into other equivalent embodiments from the present disclosure by those skilled in the art.

### [Industrial Applicability]

The present disclosure relates to a composite bulk block for manufacturing a dental prosthesis that can exhibit excellent mechanical strength and an antifouling property, to a method for manufacturing the composite bulk block, and to a dental prosthesis manufactured from the composite bulk block.

The composite bulk block according to the present disclosure has improved mechanical strength, can be used not only for inlays and onlays but also as artificial posterior teeth, and can exhibit an attachment-suppression ability against intra-oral contaminants.

## Claims

1. A composite bulk block for manufacturing a dental prosthesis, the composite bulk block comprising a glass ceramic matrix and a polymer,
wherein the glass ceramic matrix is composed of an amorphous glass matrix and a crystalline phase dispersed in the glass matrix, the crystalline phase includes at least one selected from a leucite crystalline phase and a lithium disilicate crystalline phase as a main crystalline phase,
the polymer is included in an amount of 20 to 40 percent by weight with respect to weight of the entire bulk block,
the glass ceramic matrix has an average particle size of 3 to 10µm, and
the polymer includes an amphoteric ionic group.

2. The composite bulk block of claim 1, wherein the amphoteric ionic group is derived from at least one selected from a group composed of 2-methacryloyloxyethyl phosphorylcholine (MPC), sulfobetaine methacrylate (SBMA), (1,2-dimyristoyl-sn-glycero-3-phosphatidylcholine (DMPC), 3-dimethylsulfoniopropanoate (DMSP), trigonelline, ectoine, betaine, N-(2-methacryloyloxy)ethyl-N,N-dimethylammonio propanesulfonate (SPE), N-(3-methacryloylimino)propyl-N,N-dimethylammonio propanesulfonate (SPP), carboxybetaine methacrylate (CBMA), and 3-(2'-vinyl-pyridinio)propanesulfonate (SPV).

3. The composite bulk block of claim 1, wherein the amphoteric ionic group is derived from at least one selected from a group composed of 2-methacryloyloxyethyl phosphorylcholine (MPC) and sulfobetaine methacrylate (SBMA).

4. The composite bulk block of claim 1, wherein the amphoteric ionic group is derived from 2-methacryloyloxyethyl phosphorylcholine (MPC) and sulfobetaine methacrylate (SBMA).

5. The composite bulk block of claim 4, wherein the amphoteric ionic group is derived from the use of a mixture of 2-methacryloyloxyethyl phosphorylcholine (MPC) and sulfobetaine methacrylate (SBMA) in an amount of 0.1 to 0.45 percent by weight with respect to the total composition of the composite bulk block.

6. The composite bulk block of claim 1, wherein the glass ceramic matrix is included in an amount of 74.5 to 77.9 percent by weight.

7. The composite bulk block of claim 1, wherein the polymer includes a cured product of a curable organic material selected from (meth)acrylate-based monomers and oligomers including an unsaturated double bond.

8. The composite bulk block of claim 7, wherein the curable organic material is at least one selected from a group composed of hydroxyethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyl dimethacrylate (BPDM), n-tolylglycine-glycidyl methacrylate (NTGE), polyethylene glycol dimethacrylate (PEG-DMA), and oligocarbonate dimethacrylic esters.

9. The composite bulk block of claim 1, wherein the composite bulk block for manufacturing a dental prosthesis has biaxial flexural strength of 220 to 280 MPa.

10. The composite bulk block of claim 1, wherein the glass matrix includes SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent.

11. The composite bulk block of claim 1, wherein the glass ceramic is produced by a method comprising: preparing glass powder having a maximum average particle size within 300µm by melting a glass composition including SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent, by obtaining a glass molded body of assembly size by water-quenching the molten glass, and by primarily crushing the glass molded body; performing crystallization heat treatment on the glass powder in a furnace from room temperature to a maximum temperature of 755~810°C for 30 minutes to 6 hours; producing glass ceramic powder having an average particle size within 3 to 10µm by crushing the crystallization heat-treated powder; and molding the glass ceramic powder into a predetermined shape.

12. A method for manufacturing a composite bulk block for manufacturing a dental prosthesis, the method comprising:
preparing crystallizable glass powder having a maximum average particle size within 300µm or less;
performing crystallization heat treatment on the crystallizable glass powder in a furnace from room temperature to a maximum temperature of 755~810°C for 30 minutes to 6 hours;
producing glass ceramic powder having a maximum average particle size of 3 to 10µm by crushing the crystallization heat-treated powder; and
molding a glass ceramic matrix by molding the glass ceramic powder into a predetermined shape.

13. The method of claim 12, wherein the polymer is included in an amount of 20 to 40 percent by weight in the entire composite bulk block.

14. The method of claim 12 or 13, wherein the polymer includes an amphoteric ionic group.

15. The method of claim 14, wherein the amphoteric ionic group is derived from at least one selected from a group composed of 2-methacryloyloxyethyl phosphorylcholine (MPC), sulfobetaine methacrylate (SBMA), (1,2-dimyristoyl-sn-glycero-3-phosphatidylcholine (DMPC), 3-dimethylsulfoniopropanoate (DMSP), trigonelline, ectoine, betaine, N-(2-methacryloyloxy)ethyl-N,N-dimethylammonio propanesulfonate (SPE), N-(3-methacryloylimino)propyl-N,N-dimethylammonio propanesulfonate (SPP), carboxybetaine methacrylate (CBMA), and 3-(2'-vinyl-pyridinio)propanesulfonate (SPV).

16. The method of claim 14, wherein the amphoteric ionic group is derived from at least one selected from a group composed of 2-methacryloyloxyethyl phosphorylcholine (MPC) and sulfobetaine methacrylate (SBMA).

17. The method of claim 14, wherein the amphoteric ionic group is derived from 2-methacryloyloxyethyl phosphorylcholine (MPC) and sulfobetaine methacrylate (SBMA).

18. The method of claim 17, wherein the amphoteric ionic group is derived from the use of a mixture of 2-methacryloyloxyethyl phosphorylcholine (MPC) and sulfobetaine methacrylate (SBMA) in an amount of 0.1 to 0.45 percent by weight with respect to the total composition of the composite bulk block.

19. The method of claim 12, wherein the glass ceramic matrix is included in an amount of 74.5 to 77.9 percent by weight.

20. The method of claim 12, wherein the polymer includes a cured product of a curable organic material selected from (meth)acrylate-based monomers and oligomers including an unsaturated double bond.

21. The method of claim 20, wherein the curable organic material is at least one selected from a group composed of hydroxyethyl methacrylate (HEMA), 2,2-bis[4-(2-hydroxy-3-methacryloyloxy propoxy)phenyl]propane (Bis-GMA), triethylene glycol dimethacrylate (TEGDMA), diurethane dimethacrylate (UDMA), urethane dimethacrylate (UDM), biphenyl dimethacrylate (BPDM), n-tolylglycine-glycidyl methacrylate (NTGE), polyethylene glycol dimethacrylate (PEG-DMA), and oligocarbonate dimethacrylic esters.

22. The method of claim 12, wherein the crystallizable glass powder is obtained from a glass composition including SiO₂ 69.0~75.0 wt%, Li₂O 12.0~14.0 wt%, Al₂O₃ 2.5~3.5 wt%, ZnO 0.12~0.22 wt%, K₂O 1.1~2.7 wt%, Na₂O 0.1~0.3 wt%, P₂O₅ 2.0~6.0 wt%, and a coloring agent.

23. The method of claim 22, wherein the crystallizable glass powder is obtained by melting a glass composition, obtaining a glass molded body of assembly size by water quenching the molten glass, and primarily crushing the glass molded body.

24. A prosthesis processed from the composite bulk block of claim 1, the prosthesis comprising a glass ceramic matrix and a polymer,
wherein the glass ceramic matrix is composed of an amorphous glass matrix and a crystalline phase dispersed in the glass matrix, the crystalline phase includes at least one selected from a leucite crystalline phase and a lithium disilicate crystalline phase as a main crystalline phase,
the polymer is included in an amount of 20 to 40 percent by weight with respect to weight of the entire bulk block,
the glass ceramic matrix has an average particle size of 3 to 10µm, and
the polymer includes an amphoteric ionic group.
